# FASCICULE DE BREVET EUROPEEN

(11) **EP 2 858 589 B1**
(45) Date de publication et mention de la délivrance du brevet: **06.04.2016**
(21) Numéro de dépôt: 13727196.1
(22) Date de dépôt: 06.06.2013
(51) Int. Cl.: A61C 1/06, A61C 1/00, A61B 17/16

(54) **SYSTÈME CHIRURGICAL, NOTAMMENT DENTAIRE**
CHIRURGISCHES SYSTEM, INSBESONDERE EIN ZAHNÄRZTLICHES CHIRURGISCHES SYSTEM
SURGICAL SYSTEM, IN PARTICULAR DENTAL SURGICAL SYSTEM

(30) Priorité: 11.06.2012 CH 814122012
(43) Date de publication de la demande: 15.04.2015
(73) Titulaire: Dassym SA, 2068 Hauterive (CH)
(72) Inventeur: VOILLAT, Jean-Pierre, 2857 Montavon (CH)
(74) Mandataire: P&TS SA (AG, Ltd.)
(86) Numéro de dépôt international: PCT/EP2013/061740
(87) Numéro de publication internationale: WO 2013/186123

(56) Documents cités:
- US-A- 5 235 261
- US-A1- 2006 240 382
- US-A1- 2011 266 124

## Description

### Domaine technique

La présente invention concerne un système chirurgical, notamment dentaire, comprenant un instrument chirurgical ou dentaire et un dispositif de commande de l'instrument.

### Etat de la technique

Des systèmes chirurgicaux, notamment dentaires, comportent un instrument comprenant une pièce à main, par exemple un contre-angle, qui peut être munie d'un outil, par exemple d'une fraise - et un moteur tournant qui entraîne l'outil. La pièce à main et le moteur peuvent être deux pièces séparées et reliées entre elles par des interfaces appropriées ou constituer une seule pièce. Le dispositif de commande de l'instrument est souvent lié à la chaise du patient et relié à l'instrument manuel par un raccord flexible contenant des fils électriques et des tubes de passage pour l'air et l'eau.

Les moteurs rotatifs utilisés dans ce type d'instrument à main sont de plus en plus souvent des moteurs sans collecteurs et sans balais qui ont l'avantage d'être plus robustes et de pouvoir être stérilisés complètement. Pour éviter un échauffement excessif de l'instrument, qui peut être amené à tourner à des vitesses importantes, par exemple jusqu'à 40'000 tours par minute ou davantage, des moteurs triphasés sont préférables.

Un moteur rotatif utilisé dans ce type d'instrument à main est généralement constitué d'un aimant-rotor à deux pôles et d'un bobinage-stator à trois enroulements. Afin de commuter les bobines sur une position angulaire précise du rotor pour des rotations qui partent de 0 t/mn, il est possible d'équiper le moteur rotatif avec des capteurs, par exemple des capteurs à effet Hall ou magnétorésistifs, indiquant en permanence la position du rotor à l'électronique de commande qui est généralement externe au moteur. Des solutions sans capteurs, qui sont moins précises, sont aussi connues.

Dans le domaine médical, les dimensions autorisées sont très réduites et les câbles encombrants sont à éviter. Les recherches dans ce domaine pour des nouveaux systèmes comprenant des instruments à main portent donc sur leur miniaturisation aussi bien mécanique qu'électronique, la réduction optimale des câbles de toute nature et la conservation d'une fiabilité maximale.

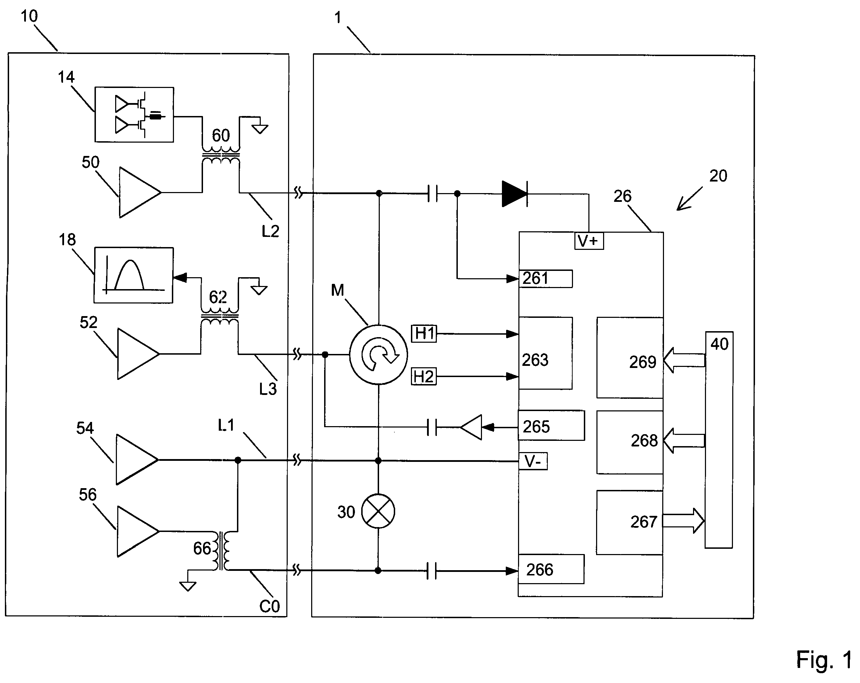

Le document WO05037124, au nom de la demanderesse, décrit un système chirurgical, notamment dentaire, dont un mode de réalisation est illustré sur la figure 1. Ce système comporte un instrument manuel 1 qui comprend un moteur rotatif M, deux capteurs à effet Hall H1 et H2 pour déterminer au moins un paramètre de fonctionnement du moteur M, et un module électronique 20.
Le dispositif de commande du moteur M comprend un premier module électronique 10 et un raccord flexible comprenant les conducteurs électriques L1, L2, L3 et C0 pour relier électriquement le premier module électronique 10 avec le deuxième module électronique 20, et donc le dispositif de commande à l'instrument. Le deuxième module électronique 20 dans l'instrument traite les signaux de sortie des capteurs H1 et H2 et les transmet au dispositif de commande 10 au travers du raccord flexible.

Le conducteur électrique L1 est le conducteur de la phase du moteur M qui correspond au neutre du point de vue haute fréquence, par exemple la phase I.

Le conducteur électrique L2 est le conducteur de la phase du moteur M, par exemple la phase II, sur lequel sont transmises la composante d'entraînement du moteur M, qui est un signal à basse fréquence, et la composante haute fréquence d'alimentation du microprocesseur 26 embarqué.

Le conducteur électrique L3 est le conducteur de la phase du moteur M, par exemple la phase III, sur lequel la composante d'entraînement du moteur M et la composante des données sont transmises depuis le microprocesseur 26 vers le module 10.

Le conducteur électrique C0 est le conducteur d'alimentation d'un accessoire 30, par exemple une lampe à incandescence ou LED, et d'acheminement des informations transmises depuis le premier module électronique 10 vers le microprocesseur 26. Les informations à l'attention du microprocesseur 26 embarqué dans l'instrument sont codées par modulation de fréquence sur ce conducteur électrique C0.

Les communications entre le premier module électronique 10 et le deuxième module électronique 20, sont donc principalement réalisées par modulation haute fréquence des conducteurs C0 et L3, notamment de la phase III du moteur. Il en découle plusieurs désavantages : il est en effet nécessaire d'utiliser des éléments magnétiques de gros volume, par exemple les deux transformateurs 62 et 66 associés respectivement à L3 et à C0, et un démodulateur 18, qui renchéri le dispositif. Le choix des types de drivers des phases moteur est en conséquence limité par le fait qu'il ne doit pas perturber les signaux haute fréquence. En outre le système est sensible aux perturbations, notamment aux perturbations dues à l'existence d'un potentiel commun entre les quatre conducteurs électriques L1, L2, L3 et C0. Le système a enfin un coût élevé.

De plus, l'utilisation de l'alimentation flottante 14 composée d'un driver PWM coûteux dans le premier module électronique 10, liée à l'alimentation 12 et permettant d'alimenter à la fois le deuxième module électronique 20, embarqué dans l'instrument à main, et le moteur M au travers du conducteur L2, et qui est potentiellement associée à la phase I (conducteur électrique L1) du moteur M implique l'utilisation d'un élément magnétique, notamment le transformateur 60 de gros volume et donc un coût élevé.

US5235261 concerne un système chirurgical, notamment dentaire qui comprend un moteur rotatif pour l'entrainement d'un outil d'un instrument. Cet instrument ne possède pas de module électronique interne au moteur (deuxième module électronique). Ce document ne décrit pas non plus un échange de messages entre un premier et un deuxième module électronique.

US2011266124 concerne un système chirurgical, notamment dentaire comprenant une électronique interne (deuxième module électronique) au moteur constituée d'un hardware fixe, exécutant des fonctions prédéfinies. En d'autres mots ce deuxième module électronique ne comprend pas de microprocesseur. Ces fonctions sont signalées à l'électronique de gestion (premier module électronique) du système au moyen de six conducteurs. Chacun de ces conducteurs porte un seul signal, donc le nombre de signaux (six) est égal au nombre des conducteurs.

US5538423 concerne une méthode et un dispositif pour contrôler des paramètres opérationnels d'un système dentaire, comprenant un instrument contenant un moteur à collecteur. Ce moteur ne comprend pas trois phases commutables, mais une seule tension variable. L'instrument ne possède pas de module électronique interne au moteur (deuxième module électronique).

EP0688539 (Bien Air) concerne une pièce à main dentaire comprenant un module électrique de commande et d'alimentation d'une lampe et d'un moteur électromagnétique sans collecteur dont le raccord comporte quatre fils d'alimentation, trois pour les trois bobines et un pour la lampe, la lampe profitant pour sa connexion sur la ligne d'alimentation négative d'un des fils (ou bornes) d'alimentation de ces trois bobines. La lampe est donc reliée aux bornes d'alimentation du moteur, ce qui peut créer des problèmes d'interférence et/ou de compatibilité électromagnétique.

EP1753360 (Bien Air) décrit un tuyau flexible reliant un instrument dentaire et une alimentation comprenant une ligne électrique supplémentaire passant par un connecteur à double fonction (conducteur de fluide et/ou d'électricité). Le conducteur supplémentaire est isolé des conducteurs pour l'alimentation d'une lampe et du moteur et peut être utilisé pour transmettre un seul signal.

Il existe donc un besoin pour un système chirurgical, notamment dentaire, qui permette de résoudre au moins un des désavantages de l'art antérieur.

Il existe aussi un besoin pour un système chirurgical, notamment dentaire, qui soit moins encombrant et plus économique que les solutions connues.

Il existe aussi un besoin pour un système chirurgical, notamment dentaire, qui soit plus fiable et qui ne présente pas des problèmes d'interférences et/ou compatibilité électromagnétique ou qui présente des problèmes d'interférences et/ou compatibilité électromagnétique réduits par rapport aux solutions connues.

### Bref résumé de l'invention

L'invention est comme revendiquée dans les revendications 1 et 13.

Un but de la présente invention est de proposer un système chirurgical, notamment dentaire, exempt au moins d'une des limitations des systèmes connus.

Un autre but de l'invention est de proposer un système chirurgical, notamment dentaire, moins encombrant et plus économique que les solutions connues.

Un autre but de l'invention est de proposer un système chirurgical, notamment dentaire, qui ne présente pas des problèmes d'interférences et/ou compatibilité électromagnétique ou qui présentes des problèmes d'interférences et/ou compatibilité électromagnétique réduits par rapport aux solutions connues.

Selon l'invention, ces buts sont atteints notamment au moyen d'un système chirurgical, notamment dentaire, comprenant :
- un instrument chirurgical, notamment dentaire, comprenant un outil
- un dispositif de commande de l'instrument comprenant un premier module électronique.
L'instrument comprend un moteur rotatif pour l'entraînement de l'outil et un deuxième module électronique.

Le moteur de l'instrument est alimenté électriquement par le dispositif de commande au travers de premiers conducteurs électriques dédiés à l'alimentation du moteur. L'instrument est alimenté et/ou commandé électriquement par le dispositif de commande au travers de deuxièmes conducteurs électriques qui avantageusement sont distincts des premiers conducteurs.

Avantageusement le système selon l'invention comprend aussi un module de gestion des deuxièmes conducteurs électriques pour envoyer sur les deuxièmes conducteurs électriques un nombre de signaux supérieur au nombre des deuxièmes conducteurs électriques.

Le système de l'invention permet donc une séparation complète entre les signaux d'alimentation du moteur, auxquels les premiers conducteurs électriques sont dédiés, et les signaux de commande et/ou d'alimentation de l'instrument et/ou d'un accessoire et/ou des signaux de synchronisation entre les modules électroniques, associés aux deuxièmes conducteurs électriques.

Dans une variante préférentielle les deuxièmes conducteurs électriques sont en nombre de deux et le module de gestion des deuxièmes conducteurs électriques envoie sur ces deuxièmes conducteurs électriques quatre signaux, notamment un signal d'alimentation du deuxième module électronique, un signal d'alimentation d'un accessoire, par exemple une lampe, un signal envoyé par le deuxième module électronique au premier module électronique portant au moins des informations concernant le moteur rotatif et des informations de gestion générale, et un signal envoyé par le premier module électronique au deuxième module électronique portant des informations concernant la commande donnée par le premier module électronique au deuxième module électronique. Donc le nombre de signaux envoyés sur les deuxièmes conducteurs électriques (quatre) est supérieur au nombre des deuxièmes conducteurs électriques (deux).

Dans une variante préférentielle le premier module électronique comprend le module de gestion. Ce module de gestion pourrait aussi être embarqué dans le deuxième module électronique ou bien être à la fois dans le premier et dans le deuxième module électronique.

Avantageusement le système selon l'invention permet la suppression des éléments magnétiques et donc un gain conséquent de place et d'argent, car aucun démodulateur n'est nécessaire dans le dispositif de commande afin de démoduler les signaux d'alimentation du moteur rotatif de l'instrument.

Avantageusement le système selon l'invention n'a pas une alimentation flottante, ni donc de bobines de découplage, ce qui permet un gain de place, de fiabilité et de réaliser une solution plus économique.

Le système selon l'invention permet aussi l'utilisation de communications digitales (numériques) avec des boucles de courant qui présentent un haut degré de sécurité et de fiabilité.

Selon l'invention il est possible d'avoir une haute densité d'informations et de fonctions sur les deuxièmes conducteurs électriques sans utiliser une modulation haute fréquence sur les premiers conducteurs électriques.

Dans une variante préférentielle le moteur rotatif du système selon l'invention est un moteur sans collecteur. Il est de préférence triphasé. Dans cette dernière variante les premiers conducteurs électriques sont au nombre de trois, un pour chacune des trois phases du moteur rotatif.

Dans une variante préférentielle les deuxièmes conducteurs électriques sont au nombre de deux.

Il est possible donc d'avoir cinq conducteurs électriques au total, dont trois dédiés exclusivement à l'alimentation du moteur rotatif de l'instrument et deux, galvaniquement totalement indépendants des phases moteur, dédiés à la commande de l'instrument et/ou à l'alimentation de l'instrument et/ou d'accessoires de l'instrument et/ou à la synchronisation entre les modules électroniques. L'accessoire de l'instrument est de préférence une lampe à incandescence ou une LED, permettant d'éclairer la zone de travail, mais peut aussi être par exemple un bouton de commande ou un capteur.

Avantageusement les signaux envoyés sur les deuxièmes conducteurs électriques comprennent un signal de synchronisme entre le premier module électronique et le deuxième module électronique et au moins deux des signaux suivants :
- un signal d'alimentation du deuxième module électronique
- un signal d'alimentation d'un accessoire, par exemple une lampe
- un signal d'alimentation d'organes ou fonctions auxiliaires
- un signal envoyé par le deuxième module électronique au premier module électronique portant au moins des informations concernant le moteur rotatif, ces informations comprenant la position instantanée du rotor du moteur rotatif, et des informations de gestion générale - un signal envoyé par le premier module électronique au deuxième module électronique portant des informations concernant la commande donnée par le premier module électronique au deuxième module électronique.

Avantageusement les signaux sur les deuxièmes conducteurs électriques peuvent être bidirectionnels : ils peuvent donc être envoyés par le premier module électronique au deuxième module électronique et/ou par le deuxième module électronique au premier module électronique. Dans une variante préférentielle le premier module électronique envoie au deuxième module électronique des signaux dans un premier temps et le deuxième module électronique envoie des signaux au premier module électronique dans un deuxième temps diffèrent du premier temps. En d'autres termes les signaux dans les deux sens ne sont pas envoyés simultanément sur les deuxièmes conducteurs.

Le module de gestion dans une variante est agencé pour envoyer sur les deuxièmes conducteurs électriques des cycles de N séquences périodiques, N étant un nombre positif.

Dans une variante préférentielle N = 4 et
- la première séquence correspond à un signal de synchronisme envoyé par le premier module électronique au deuxième module électronique
- la deuxième séquence correspond à un signal envoyé par le premier module électronique au deuxième module électronique
- la troisième séquence correspond à un signal envoyé par le deuxième module électronique au premier module électronique
- la quatrième séquence correspond à un signal d'alimentation du deuxième module électronique et/ou au signal d'alimentation d'un accessoire, par exemple une lampe et/ou au signal d'alimentation d'organes ou fonctions auxiliaires.

Avantageusement l'alimentation du deuxième module électronique par le premier module électronique est assurée seulement pendant la durée temporelle limitée, correspondant à la durée de la quatrième séquence.

Avantageusement les communications du deuxième module électronique vers le premier module électronique à travers les deuxièmes conducteurs électriques sont digitales, en boucle de courant, réalisées par activation de courants différentiés.

Dans une variante le premier module électronique assure au moins une des fonctions suivantes
- l'asservissement du moteur en général, notamment l'alimentation triphasée du moteur, notamment de son stator
- l'interprétation et la gestion des informations du deuxième module électronique
- l'asservissement de multiples interfaces et/ou organes auxiliaires, qui permettent certaines fonctionnalités additionnelles portées par le deuxième module électronique, par exemple et de façon non limitative la reconnaissance des instruments à main du système
- la gestion de la synchronisation avec le deuxième module électronique
- la communication au deuxième module électronique de messages de gestion générale, adressés aux fonctions auxiliaires telles qu'une lampe.

Dans une variante le deuxième module électronique assure au moins une des fonctions suivantes
- le calcul de l'angle ou position instantanée du rotor du moteur de l'instrument
- les communications avec le premier module, concernant notamment la position instantanée du rotor du moteur et des messages de gestion générale, adressés aux fonctions auxiliaires
- la gestion de fonctions auxiliaires, par exemple le réglage de l'intensité de la lampe.

L'invention concerne aussi un procédé pour un système chirurgical, notamment dentaire, comprenant :
un instrument chirurgical, notamment dentaire, comprenant un outil
un dispositif de commande de l'instrument comprenant un premier module électronique
l'instrument comprenant un moteur rotatif pour l'entraînement de l'outil et un deuxième module électronique
ledit procédé comprenant
   - l'alimentation du moteur rotatif par le premier module électronique du dispositif de commande au travers de premiers conducteurs électriques dédiés à l'alimentation du moteur rotatif;
   - l'alimentation et/ou la commande de l'instrument par le premier module électronique du dispositif de commande au travers de deuxièmes conducteurs électriques distincts des premiers conducteurs électriques,
les deuxièmes conducteurs électriques portant un nombre de signaux supérieur au nombre des deuxièmes conducteurs électriques.

### Brève description des figures

Des exemples de mise en oeuvre de l'invention sont indiqués dans la description illustrée par les figures annexées dans lesquelles :
La figure 1 illustre une vue synoptique d'un mode de réalisation d'un système chirurgical, notamment dentaire, connu.
La figure 2 illustre une vue synoptique d'un mode de réalisation du système chirurgical, notamment dentaire, selon l'invention.
La figure 3a illustre un exemple d'un mode de réalisation d'une trame comprenant quatre séquences de signaux envoyés sur les deuxièmes conducteurs électriques. La figure 3b illustre un exemple d'un signal de tension correspondant aux séquences de la figure 3a.
La figure 4a illustre un exemple d'un mode de réalisation de la configuration hardware permettant l'alimentation du deuxième module électronique par le premier module électronique.
Les figures 4b à 4d illustrent le signal de tension de la figure 3b par rapport aux signaux en correspondance des différents noeuds des circuits de la configuration hardware de la figure 4a.
La figure 5a illustre un exemple d'un mode de réalisation de la configuration hardware permettant la synchronisation du deuxième module électronique avec le premier module électronique réalisée par le premier module électronique.
Les figures 5b à 5d illustrent le signal de tension de la figure 3b par rapport aux signaux en correspondance des différents noeuds des circuits de la configuration hardware de la figure 5a.
Les figures 6a à 6e illustrent un exemple des signaux de communications envoyés par le premier module électronique au deuxième module électronique.
La figure 7 illustre un mode de réalisation d'un message envoyé par le premier module électronique au deuxième module électronique.
La figure 8 illustre un mode de réalisation d'un message envoyé par le deuxième module électronique au premier module électronique.
La figure 9 illustre un exemple du processus des périphériques SCI (« Serial Communications Interface ») des microprocesseurs du premier et deuxième module électronique en émission synchrone.
Les figures 10a à 10c illustrent un mode de réalisation de la relation entre le signal d'horloge (clock), les données et le courant sur les deuxièmes conducteurs électriques C1, C2 selon un mode de réalisation de l'invention.
La figure 11 illustre un mode de réalisation de la communication du deuxième module électronique avec le premier module électronique.
La figure 12 illustre un exemple de la manière dont le premier module électronique reconstruit les signaux d'horloge (clock) et des données dans une configuration lisible par son microprocesseur.
La figure 13 illustre un exemple d'agencement de transmission synchrone dans laquelle la combinaison signal d'horloge-données est mono-signal (« Manchester code »).
La figure 14 illustre un exemple de la technique PWM pour l'alimentation d'un accessoire tel qu'une lampe.
La figure 15 illustre un exemple des drivers du premier module électronique qui mettent en activité les différents états que les deuxièmes conducteurs électriques prennent.
La figure 16 illustre un exemple d'un mode de réalisation du deuxième module électronique et du moteur rotatif.
La figure 17 illustre un exemple d'un mode de réalisation des drivers du premier module électronique.

### Exemple(s) de modes de réalisation de l'invention

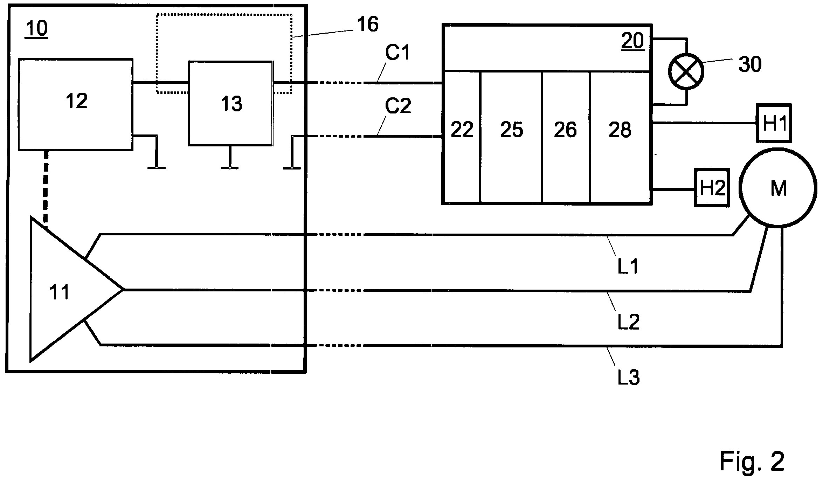

La figure 2 illustre une vue synoptique d'un mode de réalisation du système chirurgical, notamment dentaire, selon l'invention. Ce système comprend :
- un instrument chirurgical, notamment dentaire avec un moteur rotatif M pour l'entraînement d'un outil, par exemple une fraise, et un deuxième module électronique 20 dans l'instrument
- un dispositif de commande de l'instrument comprenant un premier module électronique 10.

Le moteur M comporte de préférence un nez ou accrochage non illustré destiné à recevoir une pièce à main non représentée.

L'instrument est de préférence relié à un premier module électronique 10 par un raccord flexible, par exemple un tuyau flexible non illustré attaché au moteur M. L'instrument est alimenté par exemple en eau, en air et en électricité par ce tuyau.

Dans le cas de l'instrument dentaire de la figure 2, le tuyau comprend cinq conducteurs électriques, notamment trois premiers conducteurs électriques L1, L2 et L3 et deux deuxièmes conducteurs électriques C1 et C2.

Le moteur M de l'instrument est alimenté électriquement par le dispositif de commande au travers des premiers conducteurs électriques L1 à L3 dédiés à l'alimentation du moteur. L'instrument est alimenté et commandé électriquement par le dispositif de commande au travers des deuxièmes conducteurs électriques C1 à C2 qui avantageusement sont distincts des premiers conducteurs.

Dans une variante préférentielle le moteur rotatif M du système selon l'invention est sans collecteur. Il est de préférence triphasé. Dans cette dernière variante, illustrée sur la figure 2, les premiers conducteurs électriques L1 à L3 sont au nombre de trois, un pour chacune des trois phases du moteur rotatif.

Dans la variante de la figure 2 les deuxièmes conducteurs électriques sont au nombre de deux, C1 et C2.

Il est possible donc d'avoir cinq conducteurs électriques en total, dont trois (L1 à L3) dédiés exclusivement à l'alimentation du moteur rotatif de l'instrument et deux (C1 et C2) galvaniquement totalement indépendants des phases moteur, dédiées à la commande et à l'alimentation de l'instrument et/ou à l'alimentation d'accessoires de l'instrument. L'accessoire de l'instrument est de préférence une ampoule 30, par exemple une LED, permettant d'éclairer la zone de travail, mais tout autre accessoire est aussi envisageable, par exemple un bouton poussoir ou un capteur.

Le raccord flexible reliant l'instrument dentaire au dispositif de commande peut comprendre aussi des tuyaux non illustrés pour le passage d'air et/ou de liquide. Les tuyaux pour le passage d'air et/ou de liquide comprennent de préférence une conduite d'amenée d'eau pour un spray et/ou une conduite d'amenée d'air également pour le spray et/ou une conduite d'amenée d'air de refroidissement destinée au refroidissement du moteur M et/ou une conduite de retour d'air.

Le moteur M est par exemple un moteur sans balais incluant un rotor et trois bobinages. Comme discuté, il peut s'agir par exemple d'un moteur sans collecteur triphasé comportant trois enroulements statoriques, par exemple montés en étoile.

Le moteur rotatif M peut comprendre en outre deux capteurs H1, H2 permettant de déterminer au moins un paramètre de fonctionnement du moteur. Les capteurs H1, H2 sont par exemple des capteurs analogiques à effet de Hall ou magnéto-résistifs permettant de déterminer la position angulaire instantanée du rotor du moteur. Comme illustré sur la figure 2, les deux capteurs H1, H2 sont de préférence disposés de manière à former entre eux un angle de 90°.

Avantageusement le système selon l'invention comprend aussi un module de gestion 13, qui dans l'exemple illustré est dans le premier module électronique 10, afin d'envoyer sur les deuxièmes conducteurs électriques C1, C2 un nombre de signaux supérieur au nombre des deuxièmes conducteurs électriques C1, C2.

En d'autres termes ce module de gestion 13 gère temporellement les séquences entre les modules électroniques 10, 20 en effectuant une sorte de multiplexage temporel de plusieurs signaux sur les deuxièmes conducteurs électriques C1 et C2, dans lequel ces signaux sont bidirectionnels : certains sont envoyés par le premier module électronique 10 au deuxième 20, d'autres par le deuxième module électronique 20 au premier 10. Comme discuté, dans une variante préférentielle les signaux dans les deux sens ne sont pas simultanés.

Le système de l'invention permet donc une séparation complète entre les signaux d'alimentation du moteur, auxquels les premiers conducteurs électriques L1 à L3 sont dédiés, et les signaux de synchronisation et/ou de commande et/ou d'alimentation de l'instrument et/ou d'accessoires de l'instrument, associés aux deuxièmes conducteurs électriques C1 et C2.

Avantageusement le système selon l'invention permet la suppression des éléments magnétiques et donc un conséquent gain de place et d'argent, car le démodulateur dans le dispositif de commande qui permettait de démoduler les signaux d'alimentation du moteur rotatif M de l'instrument des autres signaux n'est plus nécessaire.

Le premier module électronique 10 comprend un micro-processeur 16, une alimentation 12, un module de commande 11 des premiers conducteurs électriques L1 à L3 et le module de gestion 13 des deuxièmes conducteurs électriques C1 et C2.

Le deuxième module électronique 20 comprend un micro-processeur 26, un module d'alimentation 22, un module d'acquisition 28 des positions du moteur rotatif M ainsi qu'un module de gestion des communications numériques 25 en boucle de courant sur les deuxièmes conducteurs électriques C1 et C2, comme on le verra plus loin.

Dans la variante illustré le deuxième module électronique 20 est relié à un accessoire 30 et deux capteurs de position H1 et H2, par exemple deux capteurs à effet Hall.

Avantageusement le système selon l'invention n'a plus l'alimentation flottante 14 de la figure 1, et donc des bobines de découplage, ce qui permet un gain de place, de fiabilité et de réaliser une solution plus économique.

Avantageusement le système selon l'invention n'a pas non plus le démodulateur 18 de la figure 1, car il n'est plus nécessaire de transmettre les signaux de communications entre le premier et le deuxième modules électroniques 10, 20 en utilisant une modulation haute fréquence : en effet la présence des deuxièmes conducteurs électriques C1 à C2 rend inutile la réalisation de cette modulation sur les conducteurs L1 à L3.

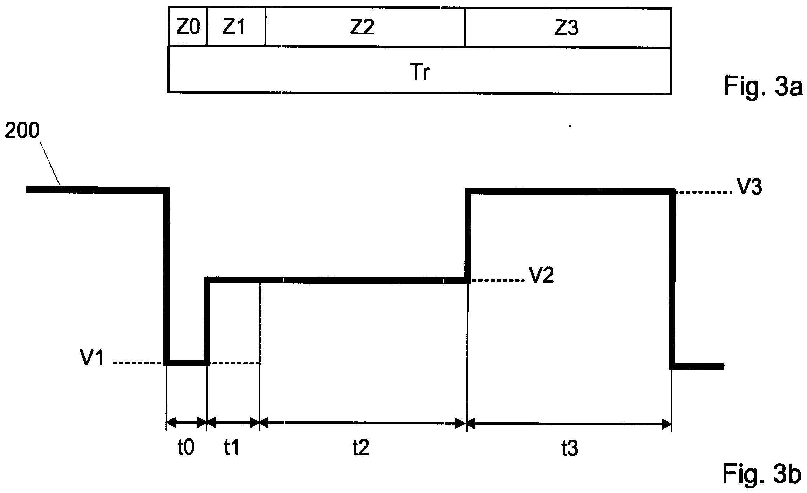

Les deuxièmes conducteurs électriques C1 et C2 peuvent travailler par cycles de N séquences périodiques, N étant un nombre positif. N séquences constituent une trame Tr. Dans la variante illustré sur la figure 3a N = 4 et les séquences sont numérotées Z0 à Z3. La figure 3b illustre un exemple d'un signal de tension 200 correspondant à la trame de la figure 3a.

Sur les deuxièmes conducteurs électriques C1 et C2 sont multiplexés le signal concernant le synchronisme entre le premier module électronique 10 et le deuxième module électronique 20 et au moins deux des suivants signaux :
- le signal d'alimentation du deuxième module électronique 20
- le signal d'alimentation d'un accessoire, par exemple une lampe 30
- le signal d'alimentation d'organes ou fonctions auxiliaires
- le signal envoyé par le deuxième module électronique 20 au premier module électronique 10 : ce signal est à haute vitesse, c'est-à-dire à au moins 500kBauds et porte des informations concernant le moteur rotatif M
- le signal envoyé par le premier module électronique 10 au deuxième module électronique 20 : ce signal est à vitesse moyenne, c'est-à-dire au moins 4800 Bauds et porte des informations concernant la commande donnée par le premier module électronique 10 au deuxième module électronique 20.

Dans la variante des figures 3a et 3b, la séquence Z0 correspond à un signal de tension V1, par exemple V1 = 0 V, qui a une durée temporelle t0, par exemple t0 = 2 µs. Ce signal est un signal de synchronisme envoyé par le premier module électronique 10 au deuxième module électronique 20.

La séquence Z1 correspond à un signal de tension V2, par exemple V2 = 5 V, qui a une durée temporelle t1, par exemple t1 = 8 µs. Ce signal dans cet exemple est envoyé par le premier module électronique 10 au deuxième module électronique 20 : ce signal est à vitesse moyenne, c'est-à-dire au moins 4800 Bauds et porte des informations concernant la commande donnée par le premier module électronique 10 au deuxième module électronique 20.

La séquence Z2 correspond à un signal de tension V2, par exemple V2 = 5 V, qui a une durée temporelle t2, par exemple t2 = 50 µs. Ce signal dans cet exemple est envoyé par le deuxième module électronique 20 au première module électronique 10 : ce signal est à haute vitesse, c'est-à-dire au moins 500 KBauds et porte des informations concernant la position du rotor du moteur M.

La séquence Z3 correspond à un signal de tension V3, par exemple V3 = 12 V, qui a une durée temporelle t3, par exemple t3 = 60 µs. Ce signal est le signal d'alimentation du deuxième module électronique 20 et/ou le signal d'alimentation d'un accessoire, par exemple une lampe 30 et/ou le signal d'alimentation d'organes ou fonctions auxiliaires.

Les valeurs de tension V1 à V3 et des plages temporelles t0 à t3 données ne sont pas limitatives et peuvent être modifiées par l'homme du métier selon les applications.

Les séquences illustrées sur la figure 3a sont aussi indicatives et il est par exemple envisageable de travailler par cycles de N séquences périodiques, N étant un nombre différent de quatre. Il est aussi possible d'utiliser la trame Tr illustrée sur la figure 3a, mais avec un ordre différent des séquences, par exemple Z2, Z3, Z0 et Z1.

Dans une variante préférentielle la séquence Z3 correspond à l'alimentation du deuxième module électronique 20 par le premier module électronique 10. Cette alimentation est assurée donc seulement pendant la durée temporelle t3, qui dans une variante préférentielle est la moitié de la durée temporelle totale de la trame Tr, correspondant à t0+t1+t2+t3. Le temps restant, le deuxième module électronique 20 doit compter sur son propre stockage d'énergie.

### Séquence Z3

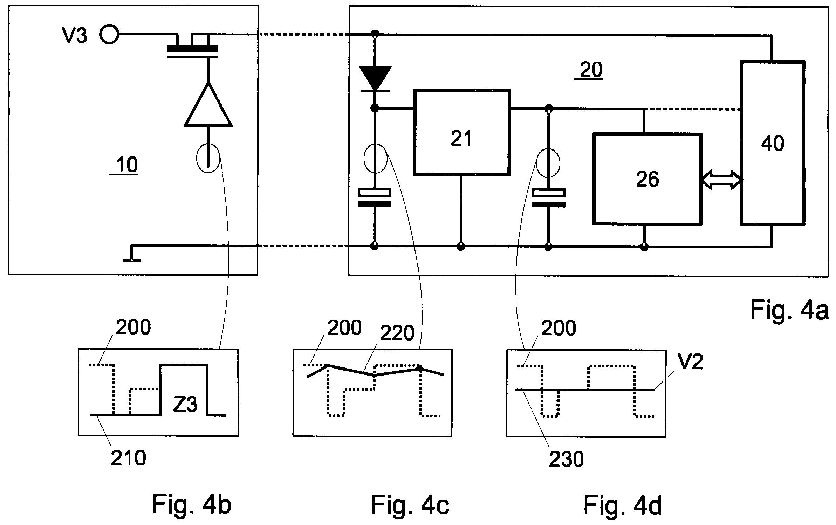

Un mode de réalisation de la configuration hardware de l'alimentation du deuxième module électronique 20 par le premier module électronique 10 est décrit sur la figure 4a.

Comme illustré sur la figure 4b, la consommation d'énergie du deuxième module électronique 20 ne s'effectue que durant la durée temporelle de la séquence Z3. Une ou plusieurs capacités du deuxième module électronique 20 permettent une accumulation d'énergie visible sur la figure 4c. Le stabilisateur 21 permet d'avoir un signal de tension constante et égale à V2, par exemple V2 = 5V, tel que visible sur la figure 4d, référence 230.

Des organes auxiliaires 40 peuvent être alimentés soit par le stabilisateur 21 dans le cas d'une consommation faible, soit directement sur les deuxièmes conducteurs électriques C1 et C2 en partant toujours du principe que la consommation d'énergie reste confinée dans la zone Z3.

### Séquence Z0

Dans une variante préférentielle les deux modules électroniques 10 et 20 sont synchronisés. Dans une variante préférentielle l'organe synchronisateur est le premier module électronique 10 (master) et l'organe synchronisé est le deuxième module électronique 20 (slave).

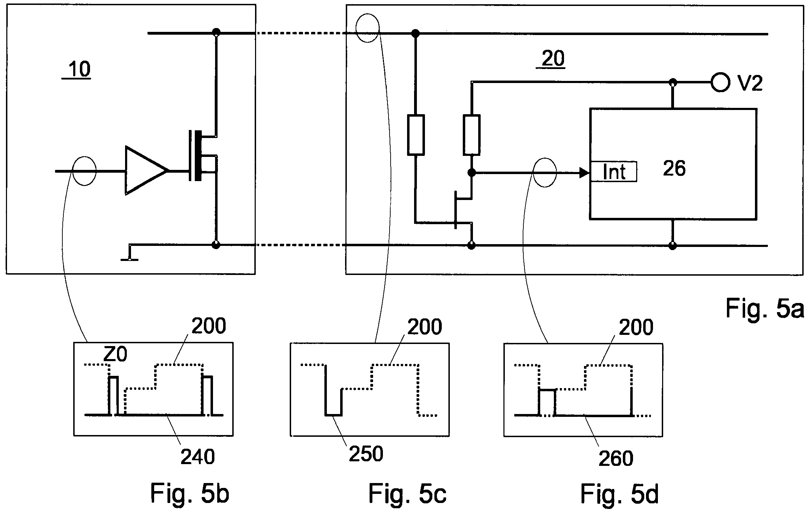

Le signal de synchronisation est donc de préférence généré par le premier module électronique 10 (master) en début de cycle, donc en correspondance de la séquence Z0, par une mise à zéro des deuxièmes conducteurs électriques C1 et C2. Ce potentiel zéro est détecté par le deuxième module électronique 20, qui provoque un signal « interrupt » sur le microprocesseur 26 embarqué, comme le montre la figure 5a.

Au signal de synchronisation 240 généré par le premier module électronique 10 et visible sur la figure 5b correspond donc une mise à zéro 250 des deuxièmes conducteurs électriques, visible sur la figure 5c. Le signal « interrupt » 260, visible sur la figure 5d, se produit à l'instant même où la tension des deuxièmes conducteurs électriques tombe à zéro. De cette façon la synchronisation entre le premier et le deuxième module n'est pas dépendante de la durée du signal de synchronisation 240.

### Séquence Z1

Comme discuté, dans une variante préférentielle les signaux émis par le premier module électronique 10 en direction du deuxième module électronique 20 (communication 10 → 20) correspondent à la séquence Z1 de la figure 3a, qui a une durée t1.

Comme discuté, il s'agit de signaux à vitesse moyenne, c'est-à-dire au moins 4800 Bauds, qui portent des informations concernant la commande donnée par le premier module électronique 10 au deuxième module électronique 20. Il s'agit donc de signaux de gestion générale de type API (« Application Programming Interface »), sans rapport direct avec la rotation du moteur rotatif M, mais adressés essentiellement aux fonctions auxiliaires, par exemple à la lampe 30.

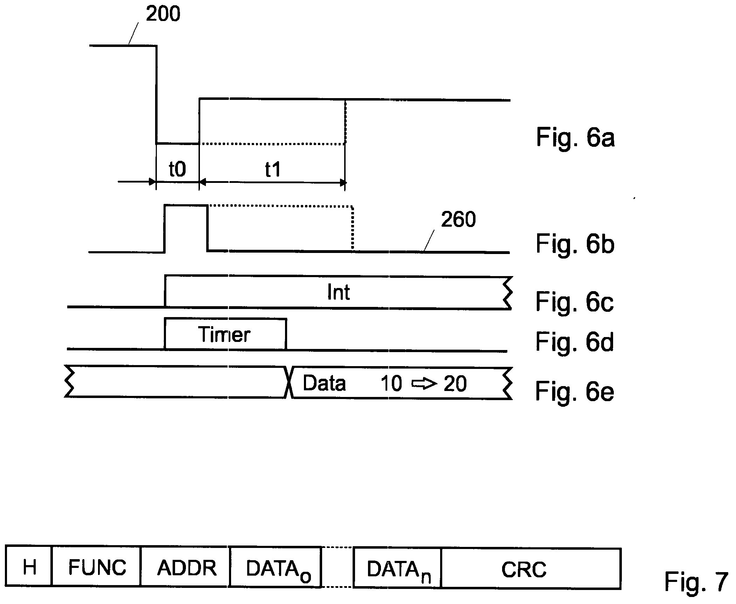

Le micro-processeur 26 du deuxième module électronique 20, dès réception du signal « interrupt » de synchronisation 260, visible sur la figure 6b, qui provoque un état d'interruption « Int » dans le microprocesseur 26 du deuxième module électronique 20, visible sur la figure 6c, engage un signal « timer », visible sur la figure 6d. Ce signal a une durée de quelques µs, par exemple de 6 µs.

Au terme de ce signal de timer, le microprocesseur 26 du deuxième module électronique 20 détermine la nature des données reçues. Une donnée "0" correspond à un signal de synchronisation 250 de courte durée, par exemple de 2 µs, et une donnée "1" correspond à un signal de synchronisation 250 de longue durée, par exemple de 10 µs.

Les données numériques des messages envoyés par le premier module électronique 10 vers le deuxième module 20 ont donc la même fréquence que la trame Tr des deuxièmes conducteurs électriques.

Si la durée totale de la trame Tr, illustrée sur la figure 3b et correspondant à t0+t1+t2+t3, est par exemple égale à 120 µs, la vitesse de transmission de ces messages est de 8,33 KBauds. Cette vitesse, bien que relativement lente, est suffisante car les ordres de gestion générale donnés au moteur M ne requièrent pas une exécution instantanée.

Le protocole de transmission des données des signaux envoyés par le premier module électronique 10 au deuxième 20 peut par exemple être le protocole API tel qu'illustré sur la figure 7 où :
- H [char] indique le début du message
- FUNC [Byte] indique la codification du message selon une table
- ADDR [Byte] est le registre des adresses
- DATAi [Byte] est la chaine de bytes
- CRC [Word] est un code de contrôle, basé par exemple su un algorithme polynomiale.

### Séquence Z2

Comme discuté la séquence Z2 correspond à un signal envoyé par le deuxième module électronique 20 au premier module électronique 10.

Ce signal porte au moins des informations concernant le moteur rotatif M, ces informations comprenant la position instantanée du rotor du moteur rotatif M. Ce signal porte aussi des informations de gestion générale similaires à celles envoyées par le premier module électronique 10 au deuxième module électronique 20.

Puisque les messages émis par le deuxième module électronique 20 en direction du premier module électronique 10 sont porteurs de la position instantanée du rotor du moteur rotatif M sans balais, position qui doit être connue en permanence par le premier module électronique 10 afin d'assurer la rotation du moteur M, ils demandent une rapidité de transmission plus élevée de celle des messages envoyés par le premier module électronique 10 au deuxième module électronique 20.

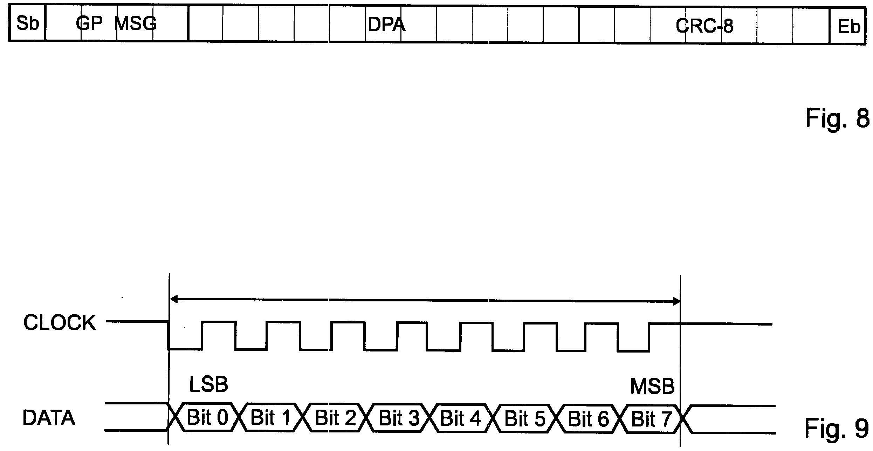

Ces communications s'effectuent durant la séquence Z2 par un message qui dans une variante préférentielle est composé par 24 bits. Un exemple d'un tel message est illustré sur la figure 8, où
- Sb (« Start bit ») est le premier bit
- GP MSG est un message de gestion générale, dont le protocole peut être différent selon les applications; dans la variante illustrée le message est composé de quatre bits
- DPA (Droite de Position Angulaire) est la position instantanée du rotor du moteur rotatif M ; dans une variante préférentielle 360° correspondent à 4096 unité d'angle
- CRC-8 (« Cyclic Redundancy Check ») est un polynôme de contrôle normalisé, par exemple CRC8 CCITT x⁸ + x² + x + 1
- Eb (« End bit ») est le dernier bit.

Comme discuté, la séquence Z2 n'engendre pas de consommation d'énergie par le deuxième module électronique 20 : il est donc possible de consommer l'énergie uniquement lors d'un transfert d'informations. Par exemple le bit "0" peut correspondre à l'absence de consommation d'énergie, notamment de courant, tandis que le bit "1" peut correspondre à une consommation de courant prédéterminée, par exemple et de façon non limitative une consommation de 50 mA.

Puisqu'il y a de préférence une communication synchrone entre le microprocesseur 16 du premier module électronique 10 et le microprocesseur 26 du deuxième module électronique 20, un signal d'horloge (« clock ») doit être associé aux données pour signaler au récepteur, notamment au premier module électronique 10, le moment de validité de la donnée transmise. D'autres solutions sans signal de « clock », mais exploitant par exemple la valeur fixe d'un premier bit d'une séquence peuvent aussi être envisagées.

La figure 9 illustre un exemple du processus des périphériques SCI (« Serial Communications Interface ») des microprocesseurs 16 et 26 en émission synchrone. Le signal de clock définit, dans le cas d'une transmission qui n'est pas continue, une fenêtre temporelle, indiquée sur la figure 9 par la flèche, qui permet de prendre en considération seulement les bits des données ou « data » qui tombent dans cette fenêtre, dans l'exemple illustré les bits 0 à 7.

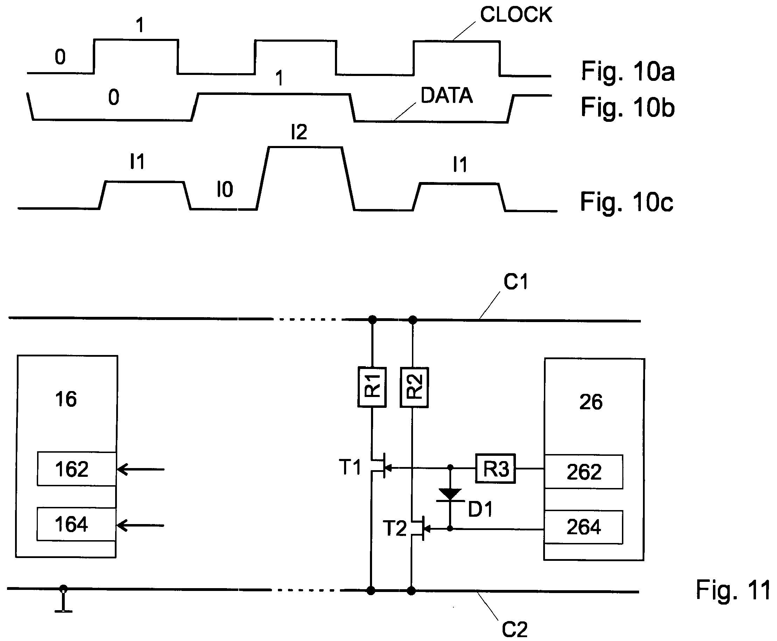

Les figures 10a à 10c illustrent un mode de réalisation de la relation entre le clock, les données et le courant sur les deuxièmes conducteurs électriques C1, C2 selon un mode de réalisation de l'invention.

Durant la séquence Z2 l'état "0" du signal clock, visible sur la figure 10a, peut interdire toute consommation sur les deuxièmes conducteurs électriques et l'état " 1 " peut provoquer deux consommations de courant différenciées 11, 12, visibles sur la figure 10c, selon l'état de la data correspondante, visible sur la figure 10b.

Donc, si le signal de clock est à zéro, la consommation de courant est toujours 10, qui dans une variante préférentielle correspond à 0 mA.

Si le signal de clock est à un, pour un bit des données égal à zéro, la consommation de courant par le deuxième module électronique 20 est 11, dans une variante préférentielle I1 = 25 mA.

Si le signal de clock est à un, pour un bit des données égal à un également, la consommation de courant par le deuxième module électronique 20 est 12, dans une variante préférentielle 12 = 50 mA.

La figure 11 illustre de façon schématique un mode de réalisation de la communication du deuxième module électronique avec le premier module électronique.

Si le signal de clock 264 émis par le microprocesseur 26 du deuxième module 20 est à l'état "0", de part la diode D1, aucun transistor T1, T2 ne peut être en conduction. S'il est à "1 " et les données 262 à "0", seul le transistor T2 conduit et provoque une consommation de courant de V2/R2. Dans une variante V2/R2 = 25 mA. Par contre, si le signal de clock 264 et les données 262 sont simultanément à "1", T1 et T2 sont en conduction. Dans ce cas, la consommation de courant est de V2/R1 + V2/R2. Dans une variante V2/R1 + V2/R2 = 50 mA.

Les transmissions par consommation de courant de la part du deuxième module électronique 20 vers le premier module électronique 10 prennent donc bien les caractéristiques de la figure 10c.

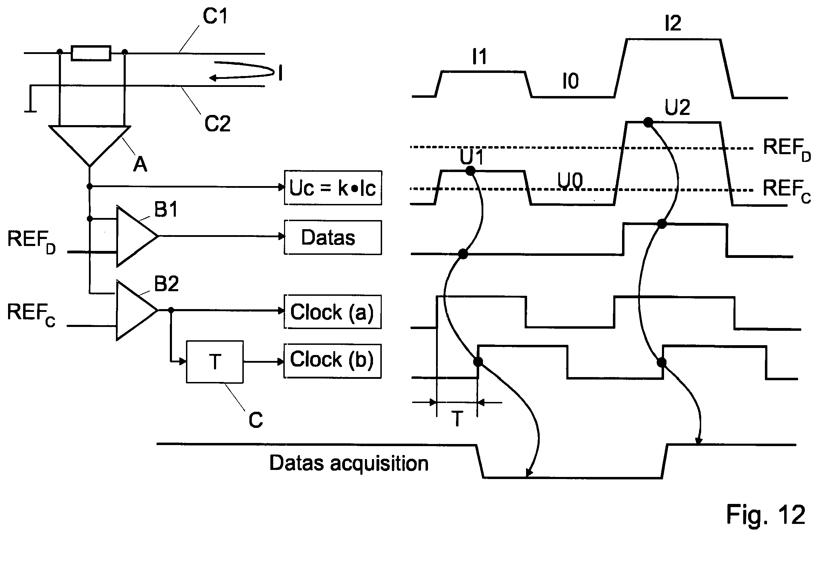

La figure 12 illustre un exemple de reconstruction des signaux de clock et des données par le premier module électronique 10 dans une configuration lisible par son microprocesseur 16.

L'amplificateur différentiel A convertit les variations de courant I des deuxièmes conducteurs électriques C1, C2 en variations de tension proportionnelles Uc = k · Ic référenciées au potentiel zéro (masse). A partir de Uc, un premier trigger B1 permet d'extraire les données d'état "1 " dès lors que Uc > REF_{D}, et un deuxième trigger B2 reforme le signal clock(a) dès lors que Uc > REF_{C}.

Afin d'avoir une acquisition correcte des données par le microprocesseur 16, un temporisateur C génère un signal clock(b) en retardant par rapport à clock(a). Un retard inférieur à 1 µs, par exemple un retard d'environ 100 ns est suffisant. De cette façon le clock agit sur un état stable des données.

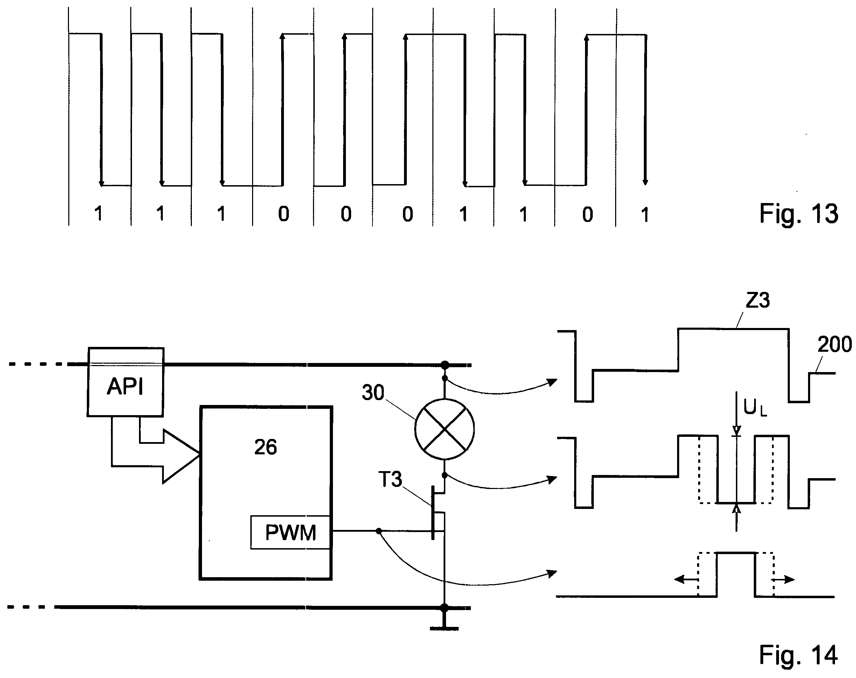

Dans les exemples illustrés sur les figures 9 à 12 le clock et les données sont deux signaux séparés, ce qui permet d'avoir un codage et décodage des signaux assez simple. Dans une autre variante, il est possible d'avoir un autre agencement de transmission synchrone dans laquelle la combinaison clock-données est mono-signal. Un exemple est donné par le « Manchester code » illustré sur la figure 13.

La gestion d'un accessoire tel qu'une lampe 30 est contrôlée par le microcontrôleur 26 du deuxième module électronique 20 selon les commandes API données par le premier module électronique 10, comme illustré sur la figure 14.

Dans une variante l'alimentation de la lampe 30 est basée sur la technique PWM (« Pulse Width Modulation ») générée par le microcontrôleur 26 du deuxième module électronique 20.

Comme discuté, dans une variante préférentielle l'alimentation de la lampe 30 n'est autorisée que durant la séquence Z3 : la fréquence du PWM est donc égale à celle des cycles des deuxièmes conducteurs électriques et sa durée d'ouverture s'étale de 0% à 50%, ou en générale à une durée d'ouverture égaie à t3.

Selon la tension lampe U_{L} demandée par l'ordre API, le microcontrôleur 26 du deuxième module électronique 20 commande une ouverture du transistor T3 de durée correspondante. Dans un exemple le rapport cyclique est de 50% maximum et la tension V2 durant Z3 est de12 Vdc, la tension lampe maximale est donc de 6 Vdc.

Des éventuels organes auxiliaires peuvent être alimentés lors de la séquence Z3 de la même manière que la lampe 30, par exemple par un deuxième PWM, ou, si leur consommation est faible, par exemple inférieure à 100mA, de la même manière que le deuxième module électronique 20.

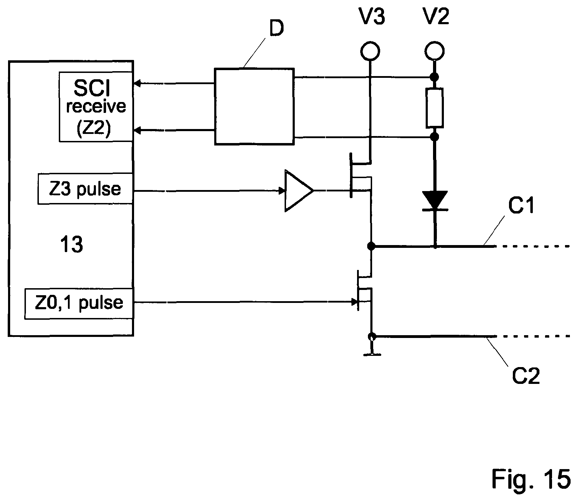

La figure 15 illustre un exemple de réalisation du module de gestion des deuxièmes conducteurs électriques qui mette en activité les différentes états que les deuxièmes conducteurs C1, C2 prennent. La référence D indique le décodeur des messages envoyés par le deuxième module 20 au premier 10 lors de la séquence Z2.

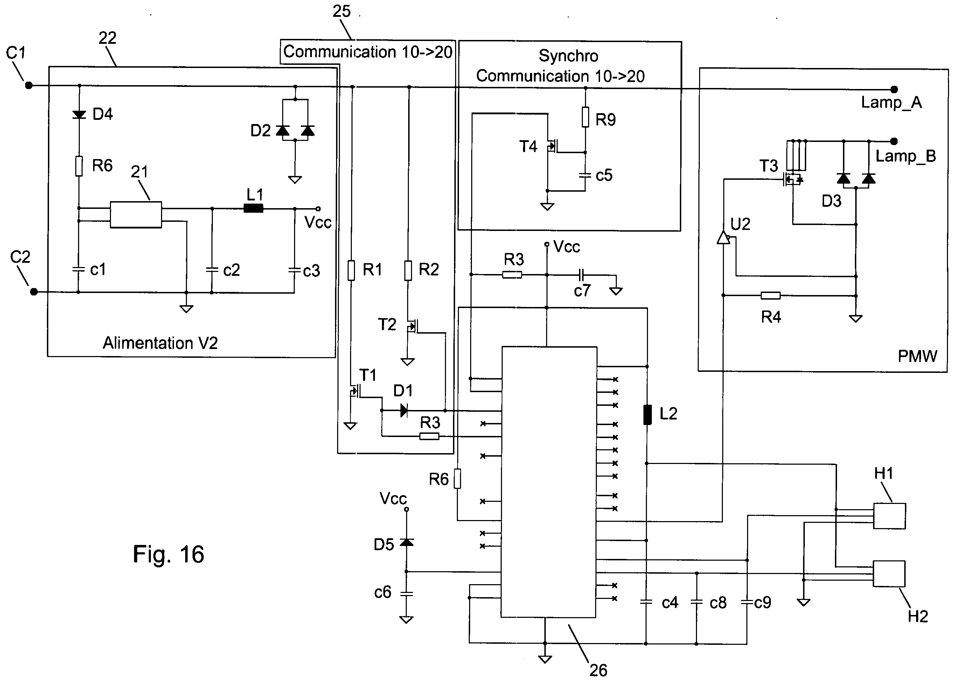

La figure 16 illustre un exemple d'un mode de réalisation du deuxième module électronique 20 et du moteur rotatif M.

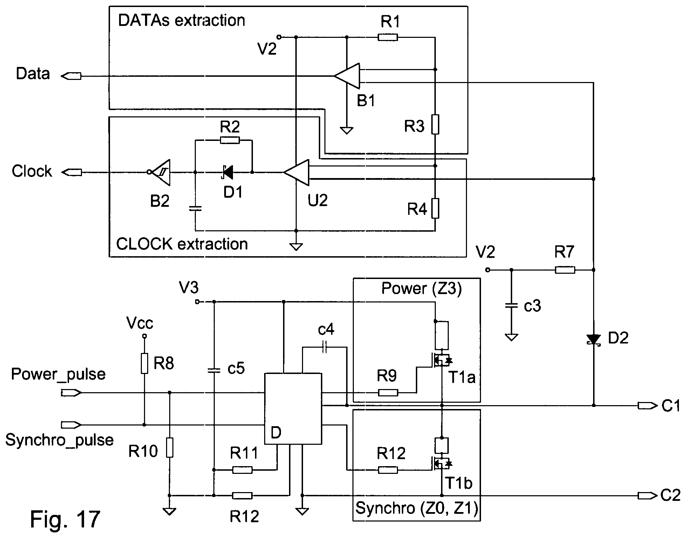

La figure 17 illustre un exemple d'un mode de réalisation des drivers du premier module électronique 10.

L'invention concerne aussi un procédé pour un système chirurgical, notamment dentaire, comprenant :
un instrument chirurgical, notamment dentaire, comprenant un outil
un dispositif de commande dudit instrument comprenant un premier module électronique 10
l'instrument comprenant un moteur rotatif M pour l'entrainement de l'outil et un deuxième module électronique 20
ledit procédé comprenant
   - l'alimentation du moteur rotatif M par le premier module électronique 10 dudit dispositif de commande au travers de premiers conducteurs électriques L1, L2, L3 dédiés à l'alimentation du moteur rotatif M;
   - l'alimentation et/ou la commande de l'instrument par le premier module électronique 10 dudit dispositif de commande au travers de deuxièmes conducteurs électriques C1, C2 distincts des premiers conducteurs électriques L1, L2, L3 ;
ces deuxièmes conducteurs électriques C1, C2 portant un nombre de signaux supérieur au nombre des deuxièmes conducteurs électriques C1, C2.

### Numéros de référence employés sur les figures

- 1: Instrument
- 10: Premier module électronique (dans le dispositif de commande)
- 11: Drivers
- 12: Module d'alimentation du premier module électronique
- 13: Module de gestion des deuxièmes conducteurs électriques
- 14: Alimentation flottante
- 16: Microprocesseur du premier module électronique
- 18: Démodulateur
- 20: Deuxième dispositif de commande (dans l'instrument chirurgical, notamment dentaire)
- 21: Stabilisateur de tension
- 22: Module d'alimentation du deuxième module électronique
- 24: Module de communication haute fréquence
- 25: Module de gestion des communications numériques « current loop »
- 26: Microprocesseur du deuxième module électronique
- 28: Module d'acquisition de la position du rotor du moteur
- 30: Accessoire, par exemple une lampe
- 40: Organes auxiliaires
- 50: Amplificateur
- 52: Amplificateur
- 54: Amplificateur
- 56: Amplificateur
- 60: Transformateur
- 62: Transformateur
- 66: Transformateur
- 162: Module pour la réception des données envoyées par le deuxième module électronique
- 164: Module pour la réception du signal de « clock » envoyé par le deuxième module électronique
- 200: Signal de tension correspondant à une trame
- 210: Signal d'apport cyclique d'énergie en correspondance de la séquence Z3
- 220: Signal d'accumulation d'énergie dans le deuxième module électronique
- 230: Signal de tension égal à V2
- 240: Signal de synchronisation dans le premier module électronique
- 250: Signal de synchronisation transmis au deuxième module électronique
- 260: Signal d'interrupt
- 261: Synchro
- 262: Module pour l'envoi des données au premier module électronique
- 263: Analog/Digital
- 264: Module pour l'envoi du signal de clock au premier module électronique
- 265: Datas outputs
- 266: Data Inputs
- 267: Auxiliaries digitales outputs
- 268: Auxiliaries digitales inputs
- 299: Auxiliaries analog/digital
- L1: Premier conducteur électrique
- L2: Premier conducteur électrique
- L3: Premier conducteur électrique
- C0: Conducteur électrique d'alimentation de la lampe
- C1: Deuxième conducteur électrique
- C2: Deuxième conducteur électrique
- M: Moteur rotatif
- H1: Premier capteur à effet Hall
- H2: Deuxième capteur à effet Hall
- Z0: Première séquence
- Z1: Deuxième séquence
- Z2: Troisième séquence
- Z3: Quatrième séquence
- Tr: Trame
- V1: Première valeur de tension
- V2: Deuxième valeur de tension
- V3: Troisième valeur de tension
- t0: Durée temporelle de la première séquence
- t1: Durée temporelle de la deuxième séquence
- t2: Durée temporelle de la troisième séquence
- t3: Durée temporelle de ia quatrième séquence
- 10: Première valeur de courant
- I1: Deuxième valeur de courant
- 12: Troisième valeur de courant
- U1: Valeur de tension proportionnelle à I1
- U2: Valeur de tension proportionnelle à 12
- U3: Valeur de tension proportionnelle à I3
- R1: Resistance
- R2: Resistance
- R3: Resistance
- D1: Diode
- T1: Transistor
- T2: Transistor
- T3: Transistor
- A: Amplificateur différentiel
- B1: Premier trigger
- B2: Deuxième trigger
- C: Temporisateur
- U_{L}: Tension de la lampe
- D: Décodeur

## Revendications

1. Système chirurgical, notamment dentaire, comprenant :
- un instrument (1) chirurgical, notamment dentaire, comprenant un outil
- un dispositif de commande dudit instrument comprenant un premier module électronique (10)
ledit instrument comprenant un moteur rotatif (M) pour l'entraînement de l'outil et un deuxième module électronique (20)
ledit moteur rotatif (M) de l'instrument étant alimenté électriquement par ledit premier module électronique (10) dudit dispositif de commande au travers de premiers conducteurs électriques (L1, L2, L3) dédiés à l'alimentation dudit moteur rotatif (M) ;
ledit instrument étant alimenté et/ou commandé électriquement par ledit premier module électronique (10) dudit dispositif de commande uniquement au travers de deuxièmes conducteurs électriques (C1, C2) distincts des premiers conducteurs électriques (L1, L2, L3) le système comprenant aussi
un module de gestion (13) desdits deuxièmes conducteurs électriques (C1, C2) pour envoyer sur lesdits deuxièmes conducteurs électriques (C1, C2) un nombre de signaux supérieur au nombre desdites deuxièmes conducteurs électriques (C1, C2).

2. Le système chirurgical, notamment dentaire, selon la revendication 1, le nombre desdits deuxièmes conducteurs électriques (C1, C2) étant égal à deux.

3. Le système chirurgical, notamment dentaire, selon la revendication 2, lesdits signaux envoyés sur lesdits deuxièmes conducteurs électriques (C1, C2) comprenant un signal de synchronisme entre ledit premier module électronique (10) et le deuxième module électronique (20) et au moins deux des signaux suivants :
- un signal d'alimentation dudit deuxième module électronique (20)
- un signal d'alimentation d'un accessoire, par exemple une lampe (30)
- un signal d'alimentation d'organes ou fonctions auxiliaires
- un signal envoyé par ledit deuxième module électronique (20) audit premier module électronique (10) portant au moins des informations concernant ledit moteur rotatif (M), ces informations comprenant la position instantanée du rotor dudit moteur rotatif (M), et des informations de gestion générale
- un signal envoyé par ledit premier module électronique (10) audit deuxième module électronique (20) portant des informations concernant la commande donnée par ledit premier module électronique (10) au deuxième module électronique (20).

4. Le système chirurgical, notamment dentaire, selon la revendication 3, ledit signal envoyé par ledit deuxième module électronique (20) audit premier module électronique (10) ayant un nombre de symboles par seconde d'au moins 500 kBauds.

5. Le système chirurgical, notamment dentaire, selon l'une des revendications 3 à 4, ledit signal envoyé par ledit premier module électronique (10) audit deuxième module électronique (20) ayant un nombre de symboles par seconde d'au moins 4800 Bauds.

6. Le système chirurgical, notamment dentaire, selon l'une des revendications 1 à 5, ledit module le gestion (13) étant agencé pour envoyer sur lesdits deuxièmes conducteurs électriques (C1, C2) des cycles de N séquences (Z0, Z1, Z2, Z3) périodiques, N étant un nombre positif.

7. Le système chirurgical, notamment dentaire, selon la revendication 6, N étant égal à quatre,
- la première séquence (Z0) correspondant à un signal de synchronisme entre ledit premier module électronique (10) et ledit deuxième module électronique 20
- la deuxième séquence (Z1) correspondant audit signal envoyé par ledit premier module électronique (10) audit deuxième module électronique (20)
- la troisième séquence (Z2) correspondant audit signal envoyé par ledit deuxième module électronique (20) audit premier module électronique (10)
- la quatrième séquence (Z3) correspondant audit signal d'alimentation dudit deuxième module électronique (20) et/ou au signal d'alimentation d'un accessoire, par exemple une lampe (30) et/ou au signal d'alimentation d'organes ou fonctions auxiliaires.

8. Le système chirurgical, notamment dentaire, selon la revendication 7, ladite quatrième séquence (Z3) ayant une durée temporelle (t3) correspondant à la moitié de la durée temporelle de l'ensemble des quatre séquences (Z0, Z1, Z2, Z3).

9. Le système chirurgical, notamment dentaire, selon l'une des revendications 7 à 8, l'alimentation dudit deuxième module électronique (20) par ledit premier module électronique (10) étant assurée seulement pendant la durée temporelle (t3) de la quatrième séquence (Z3).

10. Le système chirurgical, notamment dentaire, selon la revendication 9, ledit deuxième module électronique (20) comprenant des composants électroniques permettant de stocker l'énergie nécessaire pour être alimenté pendant la première, deuxième et troisième séquence.

11. Le système chirurgical, notamment dentaire, selon l'une des revendications 1 à 10, lesdits signaux sur lesdits deuxièmes conducteurs électriques (C1, C2) étant bidirectionnels.

12. Le système chirurgical, notamment dentaire, selon l'une des revendications 1 à 11, les communications du deuxième module électronique (20) vers le premier module électronique (10) à travers lesdits deuxièmes conducteurs électriques (C1, C2) étant en boucle de courant, par activation de courants différentiés.

13. Procédé pour un système chirurgical, notamment dentaire, comprenant :
un instrument chirurgical, notamment dentaire, comprenant un outil un dispositif de commande dudit instrument comprenant un premier module électronique (10)
ledit instrument comprenant un moteur rotatif (M) pour l'entraînement de l'outil et un deuxième module électronique (20)
ledit procédé comprenant
- l'alimentation dudit moteur rotatif (M) par ledit premier module électronique (10) dudit dispositif de commande au travers de premiers conducteurs électriques (L1, L2, L3) dédiés à l'alimentation dudit moteur rotatif (M) ;
- l'alimentation et/ou la commande dudit instrument par ledit premier module électronique (10) dudit dispositif de commande au travers de deuxièmes conducteurs électriques (C1, C2) distincts des premiers conducteurs électriques (L1, L2, L3),
lesdits deuxièmes conducteurs électriques (C1, C2) portant un nombre de signaux supérieur au nombre desdits deuxièmes conducteurs électriques (C1, C2).

14. Le procédé selon la revendication précédente, comprenant l'envoi sur lesdits deuxièmes conducteurs électriques (C1, C2) des cycles de N séquences (Z0, Z1, Z2, Z3) périodiques, N étant un nombre positif.

15. Le procédé selon la revendication 14, comprenant
- l'envoi par ledit premier module électronique (10) d'une première séquence (Z0) correspondant à un signal de synchronisme au deuxième module électronique (20)
- l'envoi par ledit premier module électronique (10) d'une deuxième séquence (Z1) correspondant audit deuxième module électronique (20)
- l'envoi par ledit deuxième module électronique (20) d'une troisième séquence (Z2) correspondant audit premier module électronique (10)
- l'envoi par ledit premier module électronique (10) d'une quatrième séquence (Z3) correspondant audit signal d'alimentation et/ou au signal d'alimentation d'un accessoire, par exemple une lampe (30) et/ou au signal d'alimentation d'organes ou fonctions auxiliaires audit deuxième module électronique (20).

## Patentansprüche

1. Chirurgisches System, insbesondere Zahnsystem, aufweisend:
- ein chirurgisches Instrument (1), insbesondere ein Zahninstrument, aufweisend ein Werkzeug;
- eine Steuervorrichtung zum Steuern des Instruments aufweisend ein erstes elektronisches Modul (10), wobei das Instrument einen Drehmotor (M) zum Antrieben des Werkzeugs und ein zweites elektronisches Modul (20) aufweist;
wobei der Drehmotor (M) des Instruments elektrisch von dem ersten elektronischen Modul (10) der Steuervorrichtung durch erste elektrische Leiter (L1, L2, L3), die für die Versorgung des Drehmotors (M) vorgesehen sind, angetrieben wird,
das Instrument wird von dem ersten elektronischen Modul (10) der Steuervorrichtung nur durch zweite elektrische Leiter (C1, C2), die sich von den ersten Leiter (L1, L2, L3) unterscheiden, angetrieben und/oder elektronisch versorgt,
das System weiter aufweisend
ein Managementmodul (13) der zweiten elektrischen Leiter (C1, C2) zum Senden auf den zweiten elektrischen Leitern (C1, C2) eine Anzahl von Signalen größer als die Anzahl der zweiten elektrischen Leiter (C1, C2).

2. Das chirurgische, insbesondere Zahn-, System nach Anspruch 1, wobei die Anzahl der zweiten elektrischen Leiter (C1, C2) gleich zwei ist.

3. Das chirurgische, insbesondere Zahn-, System nach Anspruch 2, wobei die auf den zweiten elektrischen Leitern (C1, C2) gesendeten Signale ein Zeitsignal zwischen dem ersten elektronischen Modul (10) und dem zweiten elektronischen Modul (20) un mindestens zwei der folgenden Signale aufweist:
ein Stromversorgungssignal des zweiten elektronischen Moduls (20)
ein Stromversorgungssignal eines Zubehörs, zum Beispiel einer Lampe (30)
ein Stromversorgungssignal eines Hilfsorgans oder einer Hilfsfunktion
ein Signal, das von dem zweiten elektronischen Modul (20) zu dem ersten elektronischen Modul (10) geschickt wird und das mindestens Informationen bezüglich des Drehmotors (M) trägt, wobei diese Information eine momentane Position des Rotors des Dehmotors (M) und allgemeine Informationen aufweist.

4. Das chirurgische, insbesondere Zahn-, System nach Anspruch 3, wobei das von dem zweiten elektronischen Modul (20) zu dem ersten elektronischen Modul (10) geschickte Signal eine Anzahl von Symbolen pro Sekunde von mindestens 500 kBauds aufweist.

5. Das chirurgische, insbesondere Zahn-, System nach Anspruch 3 oder 4, wobei das von dem ersten elektronischen Modul (20) zu dem zweiten elektronischen Modul (10) geschickte Signal eine Anzahl von Symbolen pro Sekunde von mindestens 4800 Bauds aufweist

6. Das chirurgische, insbesondere Zahn-, System nach einem der Ansprüche 1 bis 5, wobei das Managementmodul (13) ausgebildet ist, auf den zweiten elektrischen Leitern (C1, C2) Zyklen von N periodischen Sequenzen (Z0, Z1, Z2, Z3) zu schicken, wobei N eine positive Zahl ist.

7. Das chirurgische, insbesondere Zahn-, System nach Anspruch 6, wobei N gleich vier ist,
- die erste Sequenz (Z0) einem Zeitsignal zwischen dem ersten elektronischen Modul (10) und dem zweiten elektronischen Modul (20) entspricht,
- die zweite Sequenz (Z1) dem von dem ersten elektronischen Modul (10) zu dem zweiten elektronischen Modul (20) geschickten Signal entspricht,
- die dritte Sequenz (Z2) dem von dem zweiten elektronischen Modul (20) zu dem ersten elektronischen Modul (10) geschickten Signal entspricht,
- die vierte Sequenz (Z3) dem Stromversorgungssignal des zweiten elektronischen Moduls (20) und/oder dem Stromversorgungssignal eines Zubehörs, z.B. einer Lampe, und/oder dem Stromversorgungssignal eines Hilfsorgans oder Funktion entspricht.

8. Das chirurgische, insbesondere Zahn-, System nach Anspruch 7, wobei die vierte Sequenz (Z3) eine Zeitdauer (t3) einer halben Zeitdauer aller vier Sequenzen (Z0, Z1, Z2, Z3) hat.

9. Das chirurgische, insbesondere Zahn-, System nach Anspruch 7 oder 8, wobei die Stromversorgung des zweiten elektronischen Moduls (20) durch das erste elektronische Modul (10) nur während der Zeitdauer (t3) der vierten Sequenz (Z3) bereitgestellt wird.

10. Das chirurgische, insbesondere Zahn-, System nach Anspruch 9, wobei das zweite elektronische Modul (20) elektronische Komponenten aufweist, die das Speichern der während der ersten, zweiten und dritten Sequenz bereitzustellenden Energie erlaubt.

11. Das chirurgische, insbesondere Zahn-, System nach Anspruch 7, wobei die Signale auf den zweiten elektrischen Leitern (C1, C2) bidirektional sind.

12. Das chirurgische, insbesondere Zahn-, System nach Anspruch 1 bis 11, wobei die Kommunikation des zweiten elektronischen Moduls (20) zu dem ersten elektronischen Moduls (10) durch die zweiten elektrischen Leiter (C1, C2) in einer Stromschleife durch die Aktivierung eines differentiellen Stroms sind.

13. Verfahren für chirurgisches System, insbesondere Zahnsystem, aufweisend:
- ein chirurgisches Instrument (1), insbesondere ein Zahninstrument, aufweisend ein Werkzeug;
- eine Steuervorrichtung für das Instrument aufweisend ein erstes elektronisches Modul (10), wobei das Instrument einen Drehmotor (M) zum Antrieben des Werkzeugs und ein zweites elektronisches Modul (20) aufweist;
wobei das Verfahren aufweist:
- die Stromversorgung des Drehmotors (M) durch das erste elektronische Modul (10) der Steuervorrichtung durch die ersten elektrischen Leiter (L1, L2, L3), die für die Versorgung des Drehmotors (M) ausgebildet sind,
- die Stromversorgung und/oder Steuerung des ersten elektronischen Moduls (10) der Steuervorrichtung durch die zweiten elektrischen Leiter (C1, C2), die sich von den ersten elektrischen Leitern (L1, L2, L3) unterscheiden,
wobei die zweiten elektrischen Leiter (C1, C2) eine Anzahl von Signalen größer als die Anzahl der zweiten elektrischen Leiter (C1, C2) trägt.

14. Das Verfahren nach dem vorigen Anspruch, aufweisend Senden von Zyklen von N periodischen Sequenzen (Z0, Z1, Z2, Z3) auf den zweiten elektrischen Leitern (C1, C2), wobei N eine positive Zahl ist.

15. Das Verfahren nach Anspruch 14, aufweisend
- Senden durch das erste elektronische Modul (10) eine einem Zeitsignal entsprechende erste Sequenz (Z0) zu dem zweiten elektronischen Modul (20),
- Senden durch das erste elektronische Modul (10) eine dem zweiten elektronischen Modul (20) entsprechende zweite Sequenz (Z1),
- Senden durch das zweite elektronische Modul (10) eine dem ersten elektronischen Modul (10) entsprechende dritte Sequenz (Z2),
- Senden durch das erste elektronische Modul (10) eine vierte Sequenz (Z3), die dem Stromversorgungssignal und/oder einem Stromversorgungssignal eines Zubehörs, z.B. einer Lampe, und/oder dem Stromversorgungssignal eines Hilfsorgans oder Funktion entspricht, an das zweite elektronische Modul (20).

## Claims

1. Surgical system, in particular dental system, comprising:
- a surgical instrument (1), in particular a dental instrument, comprising a tool
- a control device controlling said instrument comprising a first electronic module (10)
said instrument comprising a rotary motor (M) for driving the tool and a second electronic module (20)
said rotary motor (M) of the instrument being electrically powered by said first electronic module (10) of said control device through first electrical conductors (L1, L2, L3) dedicated to supply said rotary motor (M);
said instrument being powered and/or electrically controlled by said first electronic module (10) of said control device only through second electrical conductors (C1, C2) distinct from the first electrical conductors (L1, L2, L3) the system also comprising
a management module (13) of said second electrical conductors (C1, C2) for sending on said second electrical conductors (C1, C2) a number of signals greater than the number of said second electrical conductors (C1, C2).

2. The surgical, in particular dental, system according to claim 1, the number of said second electrical conductors (C1, C2) being equal to two.

3. The surgical, in particular dental, system according to claim 2, said signals send on said second electrical conductors (C1, C2) comprising a clock signal between said first electronic module (10) and said second electronic module (20) and at least two of the following signals:
- a power supply signal of said second electronic module (20)
- a power supply signal of an accessory, for example a lamp (30)
- a power supply signal of auxiliary organs or functions
- a signal sent by said second electronic module (20) to said first electronic module (10), carrying at least information relating to said rotary motor (M), this information comprising the instantaneous position of the rotor of said rotary motor (M), and general management information
- a signal sent by said first electronic module (10) to said second electronic module (20) carrying information relating to the command given by said first electronic module (10) to the second electronic module (20).

4. The surgical, in particular dental, system according to claim 3, said signal sent by said second electronic module (20) to said first electronic module (10) having a number of symbols per second of at least 500 kBauds.

5. The surgical, in particular dental, system, according to one of claim 3 to 4, said signal sent by said first electronic module (10) to said second electronic module (20) having a number of symbols per second of at least 4800 Bauds.

6. The surgical, in particular dental, system according to one of claim 1 to 5, said management module (13) being arranged to send on said second electrical conductors (C1, C2) cycles of N periodic sequences (Z0, Z1, Z2, Z3), N being a positive number.

7. The surgical, in particular the dental, system according to claim 6, N being equal to four,
- the first sequence (Z0) corresponding to a clock signal between said first electronic module (10) and said second electronic module (20)
- the second sequence (Z1) corresponding to said signal sent by said first electronic module (10) to said second electronic module (20)
- the third sequence (Z2) corresponding to said signal sent by said second electronic module (20) to said first electronic module (10)
- the fourth sequence (Z3) corresponding to the power supply signal of said second electronic module signal (20) and/or to the power supply signal of an accessory, for example a lamp (30) and/or to the power supply signal of auxiliary organs or functions.

8. The surgical, in particular dental, system according to claim 7, said fourth sequence (Z3) having a time duration (t3) of an half of the time duration of all four sequences (Z0, Z1, Z2, Z3).

9. The surgical, in particular dental, system according to one of claims 7 to 8, the power supply of said second electronic module (20) by said first electronic module (10) being provided only during the time duration (t3) of the fourth sequence (Z3).

10. The surgical, in particular dental, system according to claim 9, said second electronic module (20) comprising electronic components allowing to store the energy necessary to be supplied during the first, second and third sequence.

11. The surgical, in particular dental, system according to one of claims 1 to 10, said signals on said second electrical conductors (C1, C2) being bidirectional.

12. The surgical, in particular dental, system according to one of claims 1 to 11, the communications of the second electronic module (20) to the first electronic module (10) through said second electrical conductors (C1, C2) being in current loop by activation of differential currents.

13. Method for surgical system, in particular dental system, comprising:
a surgical instrument, in particular a dental instrument, comprising a tool
a control device to said instrument comprising a first electronic module (10)
said instrument comprising a rotary motor (M) for driving the tool and a second electronic module (20)
said method comprising
- the power supply of said rotary motor (M) by said first electronic module (10) of said control device through the first electrical conductors (L1, L2, L3) dedicated to the supply of said rotary motor (M),
- the power supply and/or the control of said control device by said first electronic module (10) of said control device through second electrical conductors (C1, C2) distinct from the first electrical conductors (L1, L2, L3),
said second electrical conductors (C1, C2) carrying a number of signals greater than the number of said second electrical conductors (C1, C2).

14. The method according to the preceding claim, comprising sending on said second electrical conductors (C1, C2) cycles of N periodic sequences (Z0, Z1, Z2, Z3), N being a positive number.

15. The method of claim 14, comprising
- sending by said first electronic module (10) a first sequence (Z0) corresponding to a clock signal to the second electronic module (20)
- sending by said first electronic module (10) a second sequence (Z1) corresponding to said second electronic module (20)
- sending by said second electronic module (20) a third sequence (Z2) corresponding to said first electronic module (10)
- sending by said first electronic module (10) a fourth sequence (Z3) corresponding to said supply signal and/or to the supply signal of an accessory, for example a lamp (30) and/or to the power supply signal of auxiliary organs or functions to said second electronic module (20).
